# EUROPEAN PATENT SPECIFICATION

(11) **EP 0 455 482 B1**
(45) Date of publication and mention of the grant of the patent: **20.08.1997**
(21) Application number: 91303960.8
(22) Date of filing: 01.05.1991
(51) Int. Cl.: C12N 5/08, A61K 48/00, C12N 5/10

(54) **A subset of human progenitor cells**
Subklasse von menschlichen Vorläuferzellen
Subliguée de cellules progénitrices humaines

(30) Priority: 01.05.1990 US 517101
(43) Date of publication of application: 06.11.1991
(73) Proprietor: Becton Dickinson and Company, Franklin Lakes, New Jersey 07417-1880 (US)
(72) Inventor: Terstappen, Leon, Palo Alto, CA 94303 (US); Loken, Michael, Los Altos, CA 94022 (US)
(74) Representative: Ruffles, Graham Keith

(56) References cited:
- EP-A- 341 966
- EP-A- 451 611
- EP-A- 0 317 156
- EP-A- 0 330 191
- HAEMATOLOGICA, vol. 75, Suppl. 1, February 1990; S. SIENA et al., pp. 6-10
- BLOOD, vol. 77, no. 6, 15 March 1991; L.W. TERSTAPPEN et al., pp. 1218-1227

## Description

### Field of the Invention

This invention relates to a substantially pure subset of human progenitor cells, and more particularly relates to a substantially pure subset of progenitor cells which are CD34⁺/CD38⁻. This unique population of progenitor cells comprises pluripotent stem cells. The invention further relates to the use of such pluripotent stem cells in bone marrow transplantation and gene therapy.

### Background of the Invention

The human hematopoietic system is populated by cells of several different lineages. These cells may appear in bone marrow, the thymus, lymphatic tissue(s) and in peripheral blood. Within any specific lineage, there are a number of maturational stages. In most instances, the more immature developmental stages occur within bone marrow while the more mature and final stages of development occur in peripheral blood.

There are three major lineages: the erythroid lineage which matures into red blood cells; the myelomonocytic lineage which matures into granulocytes (including neutrophils, eosinophils and basophils) and monocytes; and the lymphoid lineage which matures into B lymphocytes and T lymphocytes. Within each lineage and between each lineage, antigens are expressed differentially on the surface and in the cytoplasm of the cells in a lineage. The expression of one or more antigens and/or the intensity of expression are used to distinguish between maturational stages within a lineage and between lineages. Loken et al. have published a series of papers which describe the maturational development of B lymphocytes (Blood, 70:1316, (1987)), the development of erythroid cells (Blood, 69:255, (1987)), and the development of neutrophils (Proc. Anal. Cellular Pathol., in press) and mono-myeloid cells J. Anal. Cellular Pathol., in press). In each of these cases, Loken et al. worked backward from the most mature stage in peripheral blood and described the changes in light scattering and antigen expression on the less differentiated cells. Distinct maturational stages could be assigned to differentiating populations of cells. Thus, for example, using B lymphocytes, Loken et al. defined four stages of development: "stage B IV" comprised the mature B lymphocytes, "stage B III" represented the immature B cell, "stage B II" represented a more immature pre-B cell and "stage B I" represented the "blast" lymphoid cell. Similar stages of development are described for each of the other lineages.

Assignment of cell to lineage and to a maturational stage within a cell lineage indicates lineage committment. There are cells, however, which are uncommitted to any lineage (i.e., the "progenitor" cell) and which, therefore, retain the ability to differentiate into each lineage. This earliest progenitor cell has been defined as the pluripotent stem cell.

In U.S. Pat. No. 4,714,680, Civin describes a differentiation antigen which is recognized by the monoclonal antibody designated My-10. In normal (i.e., non-leukemic) individuals, this antigen only is found on progenitor cells within the hemtopoietic system. Accordingly, Civin has described a population of progenitor stem cells which express the antigen recognized by My-10 (i.e., express the CD34 antigen), and has described a method of using My-10 to isolate stem cells for bone marrow transplantation. My-10 has been deposited with the American Type Culture Collection (Rockville, Maryland) as HB-8483. My-10 is commercially available from Becton Dickinson Immunocytometry Systems ("BDIS") as anti-HPCA 1.

The use of an anti-CD34 antibody, such as My-10 alone, however, is not sufficient to isolate a substantially pure population of pluripotent stem cells. As noted in Loken et al., stage I B cells (which are committed to B cell lineage) express the CD34 antigen. Thus, using an anti-CD34 monoclonal antibody alone is not sufficient to distinguish between "stem cells", as described by Civin, and the true pluripotent stem cell. Accordingly, it has not been possible to isolate a pure population of these cells.

In Haematologica, 1990, Jan - Feb, Vol. 75, Suppl. 1, pp. 6-10, Siena et al report on heterogeneity of circulating hematopoietic progenitors in cancer patients treated with high-dose cyclophosphamide and recombinant human granulocyte macrophage colony stimulating factor (rhGM-CSF). They demonstrate that CD34⁺ cells from peripheral blood possess qualitatively normal hematopoietic colony growth and high cloning efficiency similarly to marrow CD34⁺ cells. In addition, CD34⁺ cells from peripheral blood are shown to display the heterogeneous flow cytometry characteristics and differentiation antigens analogous to those from bone marrow, i.e., CD34⁺/CD33⁻, CD34⁺/CD13⁻, CD34⁺/CD38⁻, CD34⁺/CD11b, CD34⁺/DRlow⁺ cells have light scatter properties of small lymphocytes while CD34⁺/CD33⁺, CD34⁺/CD13⁺, CD34⁺/CD38⁺, CD34⁺/CD11b⁺, CD34⁺/DRhigh⁺ cells have light scatter properties of blast-like cells.

EP-A 451,611 published on 16 October 1991, and thus falling for consideration under Article 54(3) EPC, describes human hematopoietic stem cells which typically are characterised as being CD34⁺/CD10⁻/CD19⁻/CD33⁻.

The benefit of obtaining a pure population of pluripotent stem cells is most readily recognized in the field of gene therapy. Briefly, gene therapy is postulated as a treatment for specific diseases where a disease is caused by a defect in a particular gene. For example, sickle cell anemia is caused by a defect in a single gene. The red blood cells of sickle cell patients contain this defective gene which, in turn, codes for a defective form of the protein hemoglobin. The defective form results in the clinical condition of sickle cell anemia. Sickle cell anemia cannot be "cured" because the underlying defect is in the gene which is included within every cell.

Gene therapy seeks to replace or repopulate the cells of the hematopoietic system with cells that do not contain the defective gene but instead contain a "normal" " gene. Using conventional recombinant DNA techniques, a "normal" gene is isolated, placed into a vector, the vector is transfected into a cell capable of expressing the product coded for by the gene. The cell then must be placed in the patient. If, the "normal" gene product is produced, the patient is "cured" of the condition. The difficulty is that the cell must be capable of continual regeneration as well as growth and differentiation.

Kwok et al., PNAS 83:4552(1986), successfully demonstrated that gene therapy was possible using progenitor cells in dogs. Kwok et al. incorporated certain genes into the equivalent of lineage committed cells by retroviral infection using standard recombinant DNA techniques and transplanted the infected cells into the dogs. They obtained expression of the gene product(s) in cells isolated from the dogs. While these cells are capable of growth and differentiation, they are not capable of self-renewal. Thus, any "cure" would be temporary. Pluripotent stem cells, however, provide a better choice of cells in which to transfect a vector containing a "normal" gene. Pluripotent stem cells have the capability not only of differentiating into cells of every lineage but also of regeneration. Thus, establishing an unlimited supply of such cells. Pluripotent stem cells also offer the advantage that they do not recognize "self." Accordingly, incidences of graft versus host disease should be reduced. By transplanting a pluripotent stem cell, therefore cells of every type in the hematopoietic system containing the "normal" gene will continually grow. Accordingly, isolation of pluripotent stem cells would be useful not only in bone marrow transplantation but also in gene therapy.

### Summary of the Invention

This invention comprises a substantially pure population of human progenitor stem cells which are are substantially lacking in lineage committed cells. These cells comprise a substantially pure population of blast colony forming cells that also are CD34⁺ and CD38⁻. Cells which are CD34⁺/CD38⁻ may be used in bone marrow transplantation to repopulate the bone marrow of a patient whose "defective" marrow has been destroyed by means such as lethally irradiation (or other agents causing aplasia without recovery), and may be used in gene therapy wherein a gene producing a protein, enzyme or other product is inserted into the DNA of CD34⁺/CD38⁻ cells which then are transplanted into a patient's bone marrow.

CD34⁺/CD38⁻ cells may be isolated from bone marrow, blood or other tissues such as the thymus, lymphatics, spleen or liver. CD34⁺/CD38⁻ cells may be obtained from such fluids or tissues by conventional means such as flow cytometry wherein fluorescently labelled anti-CD34 and anti-CD38 antibodies are used to sort a substantially pure population of cells. Means other than flow cytometry, such as magnetic bead cell separation, may be used with monoclonal antibodies against CD34 and CD38 to select and isolate a substantially pure population of such cells.

### Brief Description of the Drawings

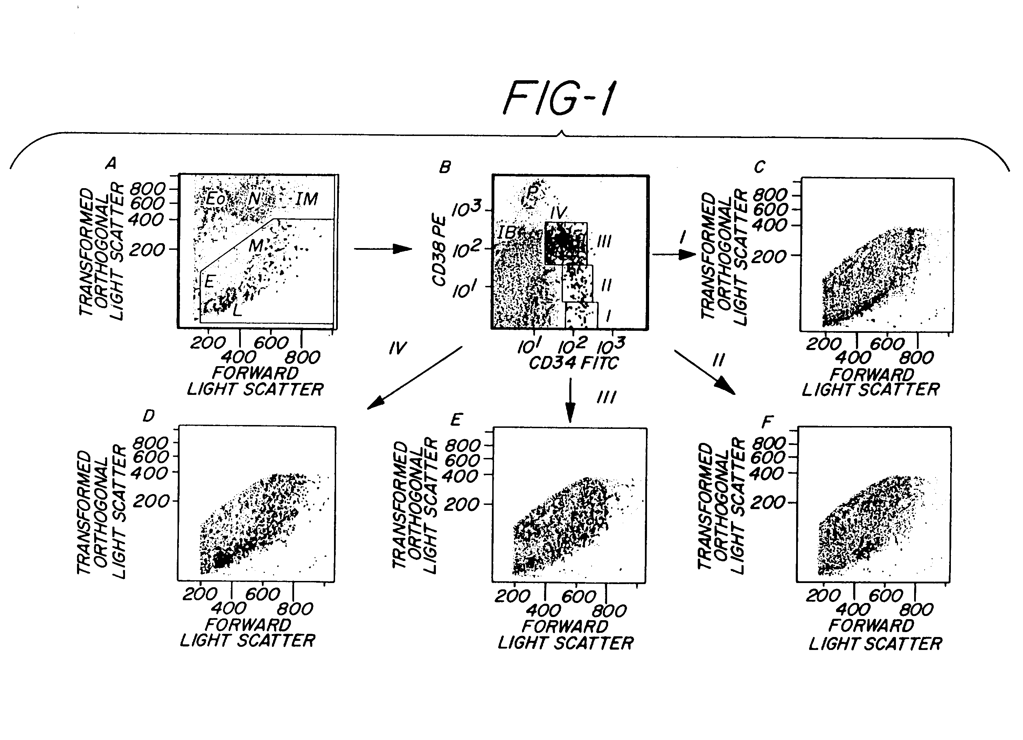

FIG. 1 comprises a series of dot plots for normal bone marrow cell aspirates labelled with anti-CD34 FITC and anti-CD38 PE monoclonal antibodies wherein (A) is a plot of transformed orthogonal light scatter vs. forward light scatter for all of the cells in the sample, (B) is a plot of PE vs. FITC log fluorescence intensities for the cells within the gate drawn in (A), (C) is a plot of transformed orthogonal light scatter vs. forward light scatter for the cells within the gate labelled I of (B), (D) is a plot of transformed orthogonal light scatter vs. forward light scatter for the cells labelled IV in (B), (E) is a plot of transformed orthogonal light scatter vs. forward light scatter for the cells within the gate labelled III in (B) and (F) is a plot transformed orthogonal light scatter vs. forward light scatter for the cells within the gate labelled II in (B);

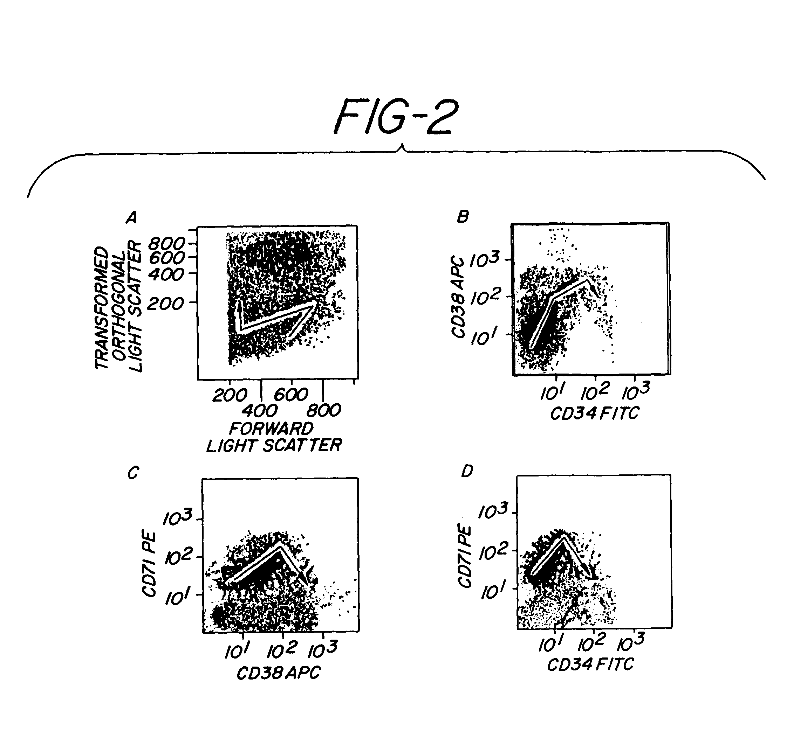

FIG. 2 comprises a series of dot plots of transformed orthogonal light scatter vs. forward light scatter (A) and log fluorescence intensities (B, C and D) of bone marrow cells in which the erythroid cells labelled with anti-CD38 APC, anti-CD34 FITC and anti-CD71 PE which are depicted as black and all other cells remain gray;

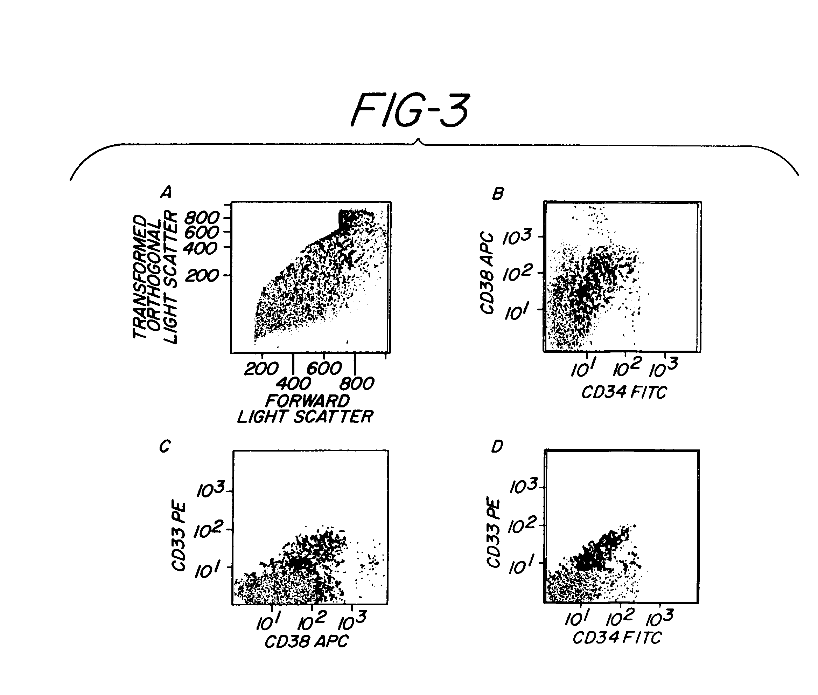

FIG. 3 comprises a series of dot plots of transformed orthogonal light scatter vs. forward light scatter (A) and log fluorescence intensities (B, C and D) of bone marrow cells in which the myelomonocytic cells labelled with anti-CD38 APC, anti-CD34 FITC and anti-CD33 PE which are depicted as black and all other cells remain gray;

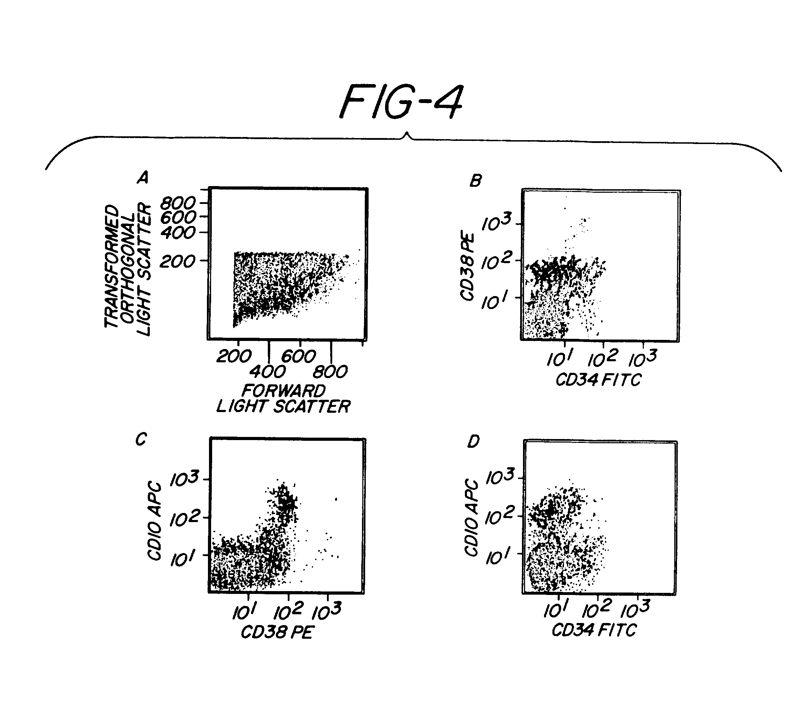

FIG. 4 comprises a series of dot plots of transformed orthogonal light scatter vs. forward light scatter (A) and log fluorescence intensities (B, C and D) of bone marrow cells in which the B lymphocyte cells labelled with anti-CD38 APC, anti-CD34 FITC and anti-CD10 APC which are depicted as black and all other cells remain gray; and

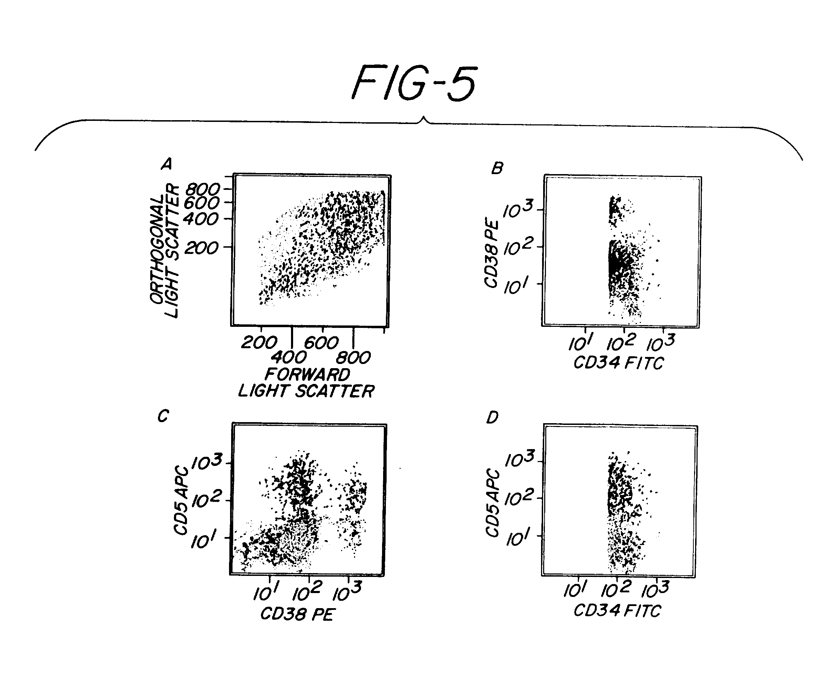

FIG. 5 comprises a series of dot plots of transformed orthogonal light scatter vs. forward light scatter (A) and log fluorescence intensities (B, C and D) of bone marrow cells in which the T lymphocyte cells labelled with anti-CD38 APC, anti-CD34 FITC and anti-CD5 APC which are depicted as black and all other cells remain gray;

### Detailed Description

The isolation and characterization of a substantially pure population of progenitor stem cells that are substantially lacking in cells committed to a specific lineage and that are CD34⁺/CD38⁻ was carried out as follows. Bone marrow aspirates were obtained from consenting normal adult volunteers. Bone marrow cell preparations were obtained using NH₄Cl lysates or density dependent centrifugation. In some instances, the cells were further enriched for CD34⁺ cells. Mature cells were depleted using anti-CD4, anti-CD8, anti-CD22 and anti-glycophorin monoclonal antibodies. These antibodies were mixed with the erythrocyte lysed bone marrow and cells binding to these antibodies were removed using anti-mouse IgG labelled magnetic microspheres. The cells not attached to the magnetic microspheres represented the enriched human progenitor cells.

In characterizing these human progenitor cells, the cells were prepared for flow cytometry. Flow cytometric analysis was performed either on a FACScan flow cytometer or a FACStar Plus cell sorter (both available from BDIS). Data acquisition was performed with FACScan Research software and FACStar Plus software (BDIS). Forward light scatter, orthogonal light scatter and three fluorescence signals were determined for each cell and stored in listmode data files. Each experiment measured approximately 30,000 cells. The analysis of the listmode data files was performed with Paint-A-Gate™ software (BDIS). (See also U.S. Pat. No. 4,845,653). For light microscopic examination, sorted cells were centrifuged for five minutes at 200g and resuspended in 100ul RPMI 1640 containing 10% fetal calf serum ("FCS"). Cytospin preparations were made on a Shandon cyto-centrifuge. Slides containing sorted cells were stained with Giemsa stain.

Erythrocyte lysed bone marrow aspirates were stained with anti-CD38 phycoerythrin ("PE") and anti-CD34 fluorescein isothiocyanate ("FITC") monoclonal antibodies (commercially available as Anti-Leu 17 PE and anti-HPCA 1 FITC, BDIS). Forward light scatter, orthogonal light scatter and two immunofluorescence parameters were determined for the bone marrow cells with a FACScan flow cytometer. One example of such an analysis is illustrated with Paint-A-Gate™ software in FIG. 1.

FIG. 1A shows the correlative display of forward and transformed orthogonal light scatter. (Transformation of the data was accomplished as set forth in commonly assigned copending application of Mickaels, Dost, Terstappen and Loken, P-1841, filed of even date herewith.) CD34⁺ cells appear black whereas all other cells are depicted gray. The position of the major cell populations are indicated with "Eo" for eosinophils, "N" for neutrophils, "IM" for immature myeloid cells, "M" for monocytes, "L" for lymphocytes and "E" for mature nucleated erythroid cells. A gate was applied on a light scattering region in which the black colored CD34⁺ cells appeared. Using this light scattering gate, an additional 30,000 cells were scanned in listmode and the correlative display of CD34 and CD38 expression is illustrated in Fig. 1B.

As in FIG. 1A, the CD34⁺ cells in FiG. 1B are depicted black and all other cells are depicted gray. The position of the previously described CD38 bright plasma cells and immature B lymphocytes are indicated with "P" and "IB" respectively. Four populations of CD34⁺ cells were distinguished based upon differential expression of CD34 and CD38 antigens. Stage P I is the smallest population, and brightly expresses the CD34 antigen but lacks the CD38 antigen. Stage P I appear in a specific light scattering region as is illustrated in FIG. 1C. Morphology of these stage P I cells showed a strikingly homogenous population of cells slightly larger than lymphocytes.

Stage P II cells were characterized by a low density expression of the CD38 antigen and a slight decrease of the CD34 antigen density in concordance with an increase in the CD38 antigen density. The stage P II cells appeared in a similar light scattering region as the stage P I cells (see FIG. 1F). The morphology of these stage P II cells is comparable with that of the cells in stage P I, although a larger heterogeneity was observed.

Stage P III cells were characterized by a large density of the CD38 antigen and an intermediate density of the CD34 antigen. With respect to light scattering properties, this population was heterogenous with a population of relatively low light scattering signals and a population with relatively large light scattering signals (FIG. 1E). This heterogeneity was confirmed by morphologic examination of the sorted cells. Blast cells of erythroid, lymphoid and myeloid origin were observed in this sorted cell fraction.

Stage P IV cells were characterized by a large density of the CD38 antigen and dim expression of the CD34 antigen. The light scattering properties of this population was even more dispersed as compared with the stage P III cells (Fig. 1D). The morphology of the cells revealed blasts of the multiple cell lineages differentiated slightly more when compared with stage P III cells.

In 10 normal bone marrow aspirates, the frequency of stage P I cells in the sample was less than 0.01% of all cells. The percentage of all CD34⁺ cells was approximately 1%.

To confirm that the CD34⁺/CD38⁻ cells indeed comprise a substantially pure population of pluripotent stem cells that are substantially lacking in lineage committed cells, cells from each of the four stages described above were examined for expression of lineage differentiation antigens.

For the erythroid lineage, bone marrow cell preparations were obtained by density dependent centrifugation. Cells were stained were anti-CD38, anti-CD34 and anti-CD71 (commercially available as Anti-Transferrin Receptor, BDIS) fluorescently labelled monoclonal antibodies. Forward light scattering, orthogonal light scatter and the three immunofluorescent parameters of the bone marrow cells were determined on a FACStar Plus cell sorter using a light scattering gate comparable to that set in FIG. 1A. FIG. 2 displays the light scatter and relative fluorescence obtained on the erythroid cells. CD71⁺ positive cells which are erythroid appear black whereas all other cells are depicted gray. Arrows in the figures indicate the maturation track of the erythroid cell lineage. The earliest recognizable erythroid cells were characterized by an expression of CD34 antigen and a large density of CD38 antigen. These early erythroid cells (erythroblast by morphology) appeared in a region concurrent with stage P IV and to a lesser extent extend into stage P III as set forth in Fig. 1. There was no expression of CD71 in stage I or in stage P II indicating that there were no erythroid committed cells in either population.

For the myelomonocytic lineages, erythrocyte lysed bone marrow aspirates were stained with anti-CD33 (commercially available as Anti-Leu M9, BDIS), anti-CD38 and anti-CD34 fluorescently labelled monoclonal antibodies. Light scatter and immunofluorescence parameters were measured as above and the results are set forth in FIG. 3. CD33 which is a marker for myelomonocytic lineages, appear black where all other cells are depicted as gray. FIG. 3D shows a population of brightly stained CD33 cells which are dimly stained with CD34 and correspond to stage P III and stage P IV cells. All CD33⁺ cells within this light scattering gate express CD38 (FIG. 3C). There was no expression of CD33 in either stage P I or stage P II indicating that there were no myelomonocytic committed cells in either population.

For the B lymphocyte lineage, low-density bone marrow cells were stained with anti-CD38, anti-CD10 (commercially available as Anti-CALLA, BDIS) and anti-CD34 fluorescently labelled monoclonal antibodies. Light scatter and immunofluorescence were again measured as above and as shown in FIG. 4. CD10⁺ cells which define the B lymphocyte lineage appear black whereas all other cells are depicted gray. FIG. 4D shows a population of brightly staining CD10⁺ cells which stain dimly with CD34 and correspond with stage P III of the progenitor cells. All CD10⁺ cells within this light scattering gate express the CD38 antigen. Again, there are no CD10⁺ positive cells in the stage P I or stage P II cells.

Finally, for the T lymphocyte lineage, low density marrow cells were stained with anti-CD38, anti-CD5 (commercially available as Anti-Leu 1, BDIS) and anti-CD34 fluorescently labelled monoclonal antibodies. Light scatter and immunofluorescence were determined as above and are shown in FIG. 5. CD5⁺ cells which is a T cell marker appear black whereas all other cells are depicted as gray. FIG. 5B shows that the CD5⁺/CD34⁺ potential T cell precursor corresponds with stage P III and stage P IV of the progenitor cells. There are no T lymphoid committed cells as defined by CD5 expression in either stage P I or in stage P II.

Apart from these studies, cells within each of the four stages were sorted into individual wells and their ability to generate blast colonies was determined. Single cells from each of the four stages were plated into 72 well plates containing liquid media for single cell culture. Each well contained a mixture of 20 ul mixture of IMDM, 2% FCS, 1% BSA, 5 X 10⁻⁵ M 2-mercaptoethanol, 600 ug/ml transferrin, 10 ug/ml soybean lecithin and antibiotics. On day 14 of incubation, recombinant human IL-3, IL-6, GM-CSF, CSF and erythropoieten ("Epo") were added to each well at a final concentration of 100U/ml with the exception of Epo which was at 2.5U/ml. All cultures were incubated in 5% CO₂ in air at 37°C in a fully humidified incubator. The plates were observed between days 24 and 34 for the appearance of blast cell colonies. Replating efficiency was determined by scoring colonies on days 7-14 after replating of the individual dispersed blast colonies in 96-well flat bottom plates under identical conditions. The results are set forth in Table 1.

**TABLE 1**

| Plating efficiency of single cells in the four identified progenitor states for colony formation as a percentage | | | | | | | |
|---|---|---|---|---|---|---|---|
| Exp | Day | Stage I | Stage II | Stage III | Stage IV | Day | ReplEff |
| 1 | 28-34 | 30.3 | 7.0 | 5.6 | 0.0 | 40-47 | nd |
| 2 | 28-34 | 25.1 | 2.8 | 0 | 1.4 | 40-47 | 74 |
| 3 | 28-34 | 25.3 | 1.8 | 2.8 | 2.4 | 40-47 | 67 |
| 4 | 28-34 | 18.8 | 8.5 | 2.1 | 0.0 | 40-47 | 100 |

As can be seen, cells in Stage P I form the greater percentage of blast colonies when compared with later stages. The replating efficiency of blast colonies was significant. This indicates that the CD34⁺/CD38⁻ cells comprise a substantially pure population of blast colony forming cells.

In another experiment, the ability to continually replate cells was examined over a course of 5 generations. In Table 2, stage P I cells were replated from single well blasts colonies. As can be see, the cells from stage P I have the ability to form blasts through at least five generations of replating. Accordingly, these cells appear to be self-renewing which is a function and characteristic of a true pluripotent stem cell.

**TABLE 2**

| Multi-Generation Colony Formation of CFU-Blast | | | | | | |
|---|---|---|---|---|---|---|
| Exp | Gen1 | Gen2 | Gen3 | Gen4 | Gen5 | Gen6 |
| 1 | 1 | 3^{a}/3^{b} | 7/4 | 61/20 | 9/9 | 1 |
| | 1 | 1/1 | 5/3 | 17/10 | 3/3 | 0 |
| | 1 | 2/2 | 4/3 | 13/10 | 2/2 | 0 |
| | 1 | 1/1 | 4/2 | 15/10 | 2/2 | 0 |
| | | | | | | |
| 2 | 1 | 1/1 | 5/2 | 13/8 | 2/2 | 0 |
| | 1 | 3/3 | 6/3 | 21/10 | 5/5 | 1 |
| | 1 | 1/1 | 3/1 | 11/5 | 2/2 | 0 |

| | | | | | | |
|---|---|---|---|---|---|---|
| a = number of colonies found | | | | | | |
| b = number of colonies replated only vital appearing colonies were replated | | | | | | |

Accordingly, to isolate a substantially pure population of human progenitor cells, a combination of anti-CD34 and anti-CD38 monoclonal antibodies are used to select those human progenitor stem cells that are CD34⁺ and CD38⁻. One method for the preparation of such a population of progenitor stem cells is to stain the cells with immunofluorescently labelled monoclonal antibodies. The cells then may be sorted by conventional flow cytometry wherein those cells that are CD34⁺ and those cells that are CD38⁻ are selected for. Upon sorting, a substantially pure population of progenitor stem cells results.

To effect gene therapy with a substantially pure population of human progenitor cells that are CD34⁺ and CD38⁻, the following method may be used to insert a gene into these progenitor cells. The method of selecting a gene, transfecting it into a stem cell and transplanting of the stem cell into a patient are not critical to the practice of the invention. The use of the substantially pure population of stem cells in the method is. For a review of the methodologies that are applicable, see Friedman, Science, 244:1275 (1989) and Lancet, June 4, 1988, p. 1271.

In order to introduce a normal gene, one must first isolate a normal gene from the cells of a donor. The cells may be isolated from tissue(s), blood or other body fluids, including bone marrow. To find a gene coding for the defective protein, DNA from the donor cells is isolated and cleaved by enzymatic digestion into segments of varying length by means know to those skilled in the art. The segments of DNA then may be inserted individually into vectors containing the appropriate regulatory sequences for expression of a gene product. The vectors then can be screened by conventional means such as Northern blotting if the sequence for the normal gene is know or the expression product can be screened by Western blotting.

Alternatively, the DNA sequence of the desired gene or the sequence of the normal protein may be known. In that case, the gene can be made by synthetic chemistries such as on a DnA Sequence (Applied Biosystems). In any case, the method of isolation or construction of the gene sequence should yield a gene that codes for the desired gene product.

Once the DNA containing the gene is prepared, the DNA can be inserted into the population of CD34⁺/CD38⁻ stem cells isolated as above. The DNA can be inserted by 1) physical methods such as coprecipitation with calcium phosphate, electroporation or microinjection (e.g., U.S. Pat. No. 4,873,191), and/or 2) the use of viral vectors such as adenoviruses, if the DNA is less than approximately 7-8 kB, or retroviruses for longer segments of DNA. In the latter case, the DNA of the retrovirus is cut with a restriction enzyme and the human DNA containing the desired sequence is inserted and ligated. The retrovirus containing the insertion them is infected into the stem cells. The stem cells then can be assayed for production of the desired protein. See, e.g., U.S. Pat. No. 4,902,783.

In general, molecular DNA cloning methods are well known in the art and are not limiting in the practice of this invention. For a further description of similar methods, see Friedmann, Science, 244:1275 (1989) and Molecular Cloning: A Laboratory Manual (2nd ed.), Cold Spring Harbor Laboratory Press (Sambrook, Fritsch and Maniatis eds., 1989).

To transplant the stem cells containing the desired gene, the cells may be introduced into the bone marrow of the patient by conventional means of bone marrow transfer. Typically, this involves the delivery of the cells by IV over a period of time. The bone marrow of the patient may be lethally irradiated prior to infusion to assure that the transplanted stem cells fully replace the existing bone marrow cells.

All publications and patent applications mentioned in this specification are indicative of the level of ordinary skill in the art to which this invention pertains. All publications and patent applications are herein incorporated by reference to the same extent as if each individual publication or patent application was specifically and individually indicated to be incorporated by reference.

## Claims

1. A substantially pure population of human cells comprising pluripotent hematopoietic stem cells that express the CD34 antigen but lack expression of the CD38 antigen and lack expression of the erythroid, myelomonocytic and lymphoid lineage associated antigens.

2. The population of claim 1 wherein the cells are derived from a hematopoietic tissue.

3. The population of cells of claim 2 wherein the cells are derived from marrow.

4. The population of cells of claim 3 wherein the cells are derived from blood.

5. A method for isolating a substantially pure population of human cells comprising pluripotent hematopoietic stem cells wherein hematopoietic cells are selected on the basis of CD34 and CD38 expression for cells that express the CD34 antigen but lack expression of the CD38 antigen.

6. The method of claim 5 wherein the stem cells are labelled with one or more labelled monoclonal antibodies that react differentially with CD34 and CD38.

7. The method of claim 5 or 6 wherein the method for selection includes means capable of discriminating between cells that react differentially with fluorescently labelled monoclonal antibodies.

8. The method of claim 7 wherein said means comprises a flow cytometer.

9. A method for altering the genome of a population of hematopoietic stem cells that express the CD34 antigen but lack expression of the CD38 antigen and lack expression of the erythroid myelomonocytic and lymphoid lineage associated antigens to insert a gene capable of producing a functional gene product comprising the steps of:
a) obtaining the gene coding for the gene product from a source; and
b) inserting the gene into one or more of the cells in the population.

10. The method of claim 9 wherein the gene isolated is inserted into a vector capable of transfecting the cells.

11. The method of claim 10 wherein the vector is a retrovirus.

12. The method of claim 9 wherein the source of the gene is DNA isolated from cells of a normal donor.

13. A substantially pure population of human stem cells comprising a population of cells that express the CD34 antigen but lack expression of the CD38 antigen, and that are thereby uncommitted to any specific lineage.

14. A substantially pure population of human stem cells comprising a population of self-renewing blast colony forming cells that are CD34⁺/CD38⁻ and lack other lineage associated antigens.

15. The method of claim 5 wherein the cells are isolated by sequential passage of cells through one or more solid support means to which one or more monoclonal antibodies are affixed thereto.

16. The method of claim 15 wherein one of said antibodies affixed to said means is an anti-CD34 monoclonal antibody.

17. The method of claim 15 wherein one of said antibodies affixed to said means is an anti-CD38 monoclonal antibody.

18. The method of claim 15 wherein said antibodies affixed to one of said means comprises monoclonal antibodies against CD38 and one or more other lineage associated antigens.

19. The population of claim 1, 13 or 14 wherein the cells are exposed to one or more growth factors.

20. The population of claim 19 wherein the growth factor is GM-CSF.

21. The method of claim 6 wherein the label is a fluorescent marker.

22. The method of claim 6 wherein the label comprises a magnetic bead and a fluorescent marker.

23. The method of claim 15 wherein one or more of the antibodies are conjugated to a label.

24. The method of claim 6 or 23 wherein the label is a magnetic bead.

25. The method of claim 15 wherein the support means comprises a magnetic bead.

26. The method of claim 25 wherein the magnetic bead is labelled with an anti-mouse immunoglobulin.

27. The method of claim 15 wherein cells are passed first through a support means having affixed thereto anti-CD34 monoclonal antibodies and then are passed through a support means having affixed thereto anti-CD38 monoclonal antibodies.

## Patentansprüche

1. Eine im wesentlichen reine Population von Humanzellen, umfassend pluripotente hämatopoietische Stammzellen, die das CD34 Antigen exprimieren, denen es aber an der Expression des CD38 Antigens fehlt, und denen es an der Expression von den mit der erythrozytären, myelomonozytären und lymphoiden Abstammungslinie assoziierten Antigenen fehlt.

2. Population nach Anspruch 1, wobei die Zellen von einem hämatopoietischen Gewebe abstammen.

3. Population von Zellen nach Anspruch 2, wobei die Zellen vom Mark abstammen.

4. Population von Zellen nach Anspruch 3, wobei die Zellen vom Blut abstammen.

5. Verfahren zum Isolieren einer im wesentlichen reinen Population von Humanzellen, umfassend pluripotente hämatopoietische Stammzellen, wobei hämatopoietische Zellen auf der Basis der CD34 und CD38 Expression für Zellen ausgewählt sind, die das CD34 Antigen exprimieren, denen es aber an der Expression des CD38 Antigens fehlt.

6. Verfahren nach Anspruch 5, wobei die Stammzellen mit einem oder mehreren markierten monoklonalen Antikörpern markiert sind, die unterschiedlich mit CD34 und CD38 reagieren.

7. Verfahren nach Anspruch 5 oder 6, wobei das Verfahren für Selektion Mitteln umfaßt, die dazu fähig sind, zwischen Zellen zu unterscheiden, die unterschiedlich mit fluoreszierend markierten monoklonalen Antikörpern reagieren.

8. Verfahren nach Anspruch 7, wobei das Mittel ein Fließzytometer umfaßt.

9. Verfahren zum Ändern des Genoms einer Population von hämatopoietischen Stammzellen, die das CD34 Antigen exprimieren, denen es aber an der Expression des CD38 Antigens fehlt, und denen es an der Expression der mit der erythrozytären, myelomonozytären und lymphoiden Abstammungslinie assoziierten Antigenen fehlt, unter Einfügen eines Gens, das sich dazu eignet, ein funktionelles Genprodukt herzustellen, umfassend die Stufen:
a) Erhalten des für das Genprodukt kodierenden Gens aus einer Quelle; und
b) Einfügen des Gens in eine oder mehrere der Zellen in der Population.

10. Verfahren nach Anspruch 9, wobei das isolierte Gen in einen Vektor eingefügt wird, der sich für die Transfektion von Zellen eignet.

11. Verfahren nach Anspruch 10, wobei der Vektor ein Retrovirus ist.

12. Verfahren nach Anspruch 9, wobei die Quelle des Gens aus Zellen eines normalen Donors isolierte DNA ist.

13. Eine im wesentlichen reine Population von Humanstammzellen, umfassend eine Population von Zellen, die das CD34 Antigen exprimieren, denen es aber an der Expression des CD38 Antigens mangelt, und die dadurch nicht an eine spezifische Abstammungslinie gebunden sind.

14. Eine im wesentlichen reine Population von Humanstammzellen, umfassend eine Population von sich selbst erneuernden Blastkolonie bildenden Zellen, die CD34⁺/CD38⁻ sind, und denen es an mit anderer Abstammungslinie assoziierten Antigenen mangelt.

15. Verfahren nach Anspruch 5, wobei die Zellen durch sequentielle Passage von Zellen durch ein oder mehrere feste Trägermittel, an die ein oder mehrere monoklonale Antikörper angefügt sind, isoliert werden.

16. Verfahren nach Anspruch 15, wobei einer der an die Mittel angehefteten Antikörper ein Anti-CD34 monoklonaler Antikörper ist.

17. Verfahren nach Anspruch 15, wobei einer der an die Mittel angehefteten Antikörper ein Anti-CD38 monoklonaler Antikörper ist.

18. Verfahren nach Anspruch 15, wobei die an eines der Mittel angehefteten Antikörper monoklonale Antikörper gegen CD38 und ein oder mehrere mit anderer Verbindungslinie assoziierte Antigene umfassen.

19. Population nach Anspruch 1, 13 oder 14, wobei die Zellen ein oder mehreren Wachstumsfaktoren ausgesetzt sind.

20. Population nach Anspruch 19, wobei der Wachstumsfaktor GM-CSF ist.

21. Verfahren nach Anspruch 6, wobei die Markierung ein Fluoreszenzmarker ist.

22. Verfahren nach Anspruch 6, wobei die Markierung eine magnetische Kugel und einen Fluoreszenzmarker umfaßt.

23. Verfahren nach Anspruch 15, wobei ein oder mehrere der Antikörper an eine Markierung konjugiert sind.

24. Verfahren nach Anspruch 6 oder 23, wobei die Markierung eine Magnetkugel ist.

25. Verfahren nach Anspruch 15, wobei das Trägermittel eine Magnetkugel umfaßt.

26. Verfahren nach Anspruch 25, wobei die Magnetkugel mit einem Anti-Maus-Immunglobulin markiert ist.

27. Verfahren nach Anspruch 15, wobei die Zellen zuerst durch ein Trägermittel mit daran angehefteten Anti-CD34 monoklonalen Antikörpern geleitet werden und anschließend durch ein Trägermittel mit daran angehefteten Anti-CD38 monoklonalen Antikörpern geleitet werden.

## Revendications

1. Population pratiquement pure de cellules humaines comprenant des cellules souches hématopoïétiques pluripotentes qui expriment l'antigène CD34 mais sont déficientes en expression de l'antigène CD38 et sont déficientes en expression des antigènes associés aux lignées érythrocytaires, myélomonocytaires et lymphoïdes.

2. Population de la revendication 1 dans laquelle les cellules proviennent d'un tissu hématopoïétique.

3. Population de cellules de la revendication 2 dans laquelle les cellules proviennent de moelle.

4. Population de cellules de la revendication 3 dans laquelle les cellules proviennent du sang.

5. Procédé d'isolement d'une population pratiquement pure de cellules humaines comprenant des cellules souches hématopoïétiques pluripotentes, dans lequel des cellules hématopoïétiques sont sélectionnées sur la base de l'expression du CD34 et du CD38 pour des cellules qui expriment l'antigène CD34 mais sont déficientes en expression de l'antigène CD38.

6. Procédé de la revendication 5 dans lequel les cellules souches sont marquées par un ou plusieurs anticorps monoclonaux marqués qui réagissent de façon différentielle avec le CD34 et avec le CD38.

7. Procédé de la revendication 5 ou 6 dans lequel le procédé employé pour la sélection comprend un dispositif capable de faire la différence entre des cellules qui réagissent de façon différentielle avec des anticorps monoclonaux marqués de manière fluorescente.

8. Procédé de la revendication 7 dans lequel ledit dispositif comprend un cytomètre en flux.

9. Procédé d'altération du génome d'une population de cellules souches hématopoïétiques qui expriment l'antigène CD34 mais sont déficientes en expression de l'antigène CD38 et sont déficientes en expression des antigènes associés aux lignées érythrocytaires, myélomonocytaires et lymphoïdes, afin d'insérer un gène capable de produire un produit génique fonctionnel, comprenant les étapes consistant:
a) à obtenir le gène codant pour le produit génique à partir d'une source; et
b) à introduire le gène dans l'une ou plusieurs des cellules présentes dans la population.

10. Procédé de la revendication 9 dans lequel le gène isolé est introduit dans un vecteur capable de transfecter les cellules.

11. Procédé de la revendication 10 dans lequel le vecteur consiste en un rétrovirus.

12. Procédé de la revendication 9 dans lequel la source du gène consiste en de l'ADN isolé de cellules d'un donneur normal.

13. Population pratiquement pure de cellules souches humaines comprenant une population de cellules qui expriment l'antigène CD34 mais sont déficientes en expression de l'antigène CD38, et qui sont de ce fait non engagées dans une quelconque lignée particulière.

14. Population pratiquement pure de cellules souches humaines comprenant une population de cellules capables de former des colonies de blastes et de s'autorenouveler, qui sont CD34⁺/CD38⁻ et sont déficientes en antigènes associés à d'autres lignées.

15. Procédé de la revendication 5 dans lequel les cellules sont isolées par passage successif de cellules à travers un ou plusieurs dispositifs de support solide auxquels un ou plusieurs anticorps monoclonaux sont fixés.

16. Procédé de la revendication 15 dans lequel un desdits anticorps fixés au(x)dit(s) dispositif(s) est un anticorps monoclonal anti-CD34.

17. Procédé de la revendication 15 dans lequel un desdits anticorps fixés au(x)dit(s) dispositif(s) est un anticorps monoclonal anti-CD38.

18. Procédé de la revendication 15 dans lequel lesdits anticorps fixés à l'un desdits dispositifs comprennent des anticorps monoclonaux contre le CD38 et un ou plusieurs antigènes associés à d'autres lignées.

19. Population de la revendication 1, 13 ou 14 dans laquelle les cellules sont exposées à un ou plusieurs facteurs de croissance.

20. Population de la revendication 19 dans laquelle le facteur de croissance est le GM-CSF.

21. Procédé de la revendication 6 dans lequel le marqueur est un marqueur fluorescent.

22. Procédé de la revendication 6 dans lequel le marqueur comprend une bille magnétique et un marqueur fluorescent.

23. Procédé de la revendication 15 dans lequel un ou plusieurs des anticorps sont conjugués à un marqueur.

24. Procédé de la revendication 6 ou 23 dans lequel le marqueur est une bille magnétique.

25. Procédé de la revendication 15 dans lequel le dispositif de support comprend une bille magnétique.

26. Procédé de la revendication 25 dans lequel la bille magnétique est marquée par une immunoglobuline anti-souris.

27. Procédé de la revendication 15 dans lequel on fait passer des cellules, d'abord à travers un dispositif de support auquel sont fixés des anticorps monoclonaux anti-CD34, et ensuite à travers un dispositif de support auquel sont fixés des anticorps monoclonaux anti-CD38.
